# EUROPEAN PATENT SPECIFICATION

(11) **EP 3 123 985 B1**
(45) Date of publication and mention of the grant of the patent: **24.02.2021**
(21) Application number: 16189783.0
(22) Date of filing: 12.03.2014
(51) Int. Cl.: A61F 2/966, A61F 2/848

(54) **DELIVERY SYSTEM FOR EXPANDABLE STENTS**
ABGABESYSTEM FÜR EXPANDIERBARE STENTS
SYSTÈME D'ADMINISTRATION POUR DES ENDOPROTHÈSES EXTENSIBLES

(30) Priority: 13.03.2013 US 201313801905
(43) Date of publication of application: 01.02.2017
(62) Divisional of application: 14159030.7
(73) Proprietor: DePuy Synthes Products, LLC, Raynham, MA 02767 (US)
(72) Inventor: GIRNARY, Hussein H., Miami, FL 33130 (US); SOTODELVALLE, Ariel, Hialeah, FL 33015 (US); LORENZO, Juan A., Davie, FL 33328 (US); FORSYTHE, Peter, Miami, FL 33130 (US)
(74) Representative: Carpmaels & Ransford LLP

(56) References cited:
- EP-A1- 1 400 219
- US-A1- 2007 255 385
- US-A1- 2009 306 761
- US-A1- 2011 301 690

## Description

### BACKGROUND OF THE INVENTION

This invention relates generally to devices for interventional therapeutic treatment or vascular surgery for treatment of defects in the vasculature, and more particularly concerns a system for delivering a self-expanding stent to a treatment site in a vasculature of a patient.

Stents, which are tubular reinforcements inserted into a blood vessel to provide an open path within the blood vessel, have been widely used in intravascular angioplasty treatment of occluded cardiac arteries. In such applications, the stent is inserted after an angioplasty procedure or the like in order to prevent restenosis of the artery. In these applications, the stents are often deployed by use of inflatable balloons, or mechanical devices which force the stent open, thereby reinforcing the artery wall and provide a clear through-path in the center of the artery after the angioplasty procedure to prevent restenosis.

While such procedures may be useful in certain aspects of vascular surgery in which vasoocclusive devices are used, the weakness of the vasculature and the tortuosity of the neurovasculature places limits on the applicability of such stents in procedures to repair neurovascular defects. Furthermore, the use of placement techniques, such as balloons or mechanical expansions of the type often found to be useful in cardiac surgery, are relatively less useful in vasoocclusive surgery, particularly when tiny vessels, such as those found in the brain, are to be treated. Hence, those skilled in the art have recognized a need for a stent compatible with techniques in vasoocclusive treatment of neurovascular defects that provides selective reinforcement in the vicinity of a neurovascular defect, while avoiding any unnecessary trauma or risk of rupture to the blood vessel.

An expandable stent and delivery system is known that includes an expandable stent having proximal and distal anchor members mounted on proximal and distal legs extending proximally and distally from the stent. The proximal and distal anchor members of the expandable stent are mounted in gaps formed between proximal, intermediate and distal cylindrical members disposed on and spaced apart along an elongated core member. However, pushing the device distally in a catheter from the proximal end of the device is not optimal, because application of force in a distal direction on the proximal end of the stent can axially compress the stent, and can cause the stent to expand radially. Likewise, retracting the device proximally may not be optimal either, because application of force in a proximal direction on the distal end of the stent also can axially compress the stent, and can cause the stent to expand radially.
EP1400219A1 relates to an expandable stent and delivery system, for enhancing luminal dilation of a blood vessel and treating aneurysms, includes proximal, intermediate and distal cylindrical members disposed on and spaced apart along an elongated core member such that first and second gaps are formed. The expandable stent includes anchor members which align with the gaps. The expandable stent is mounted on the intermediate cylindrical member, and the anchor members are disposed within the gaps thereby locking the stent onto the core member.

It would be desirable to provide a delivery system for expandable stents that offers the flexibility of engaging the device at a desired proximal, distal or other location anywhere in between, for pushing and/or pulling the device proximally or distally as desired. The present invention meets these and other needs.

### SUMMARY OF THE INVENTION

Briefly and in general terms, the present invention provides for a system for delivering and releasing a self-expanding stent to a treatment site in a patient's vasculature that allows the self-expanding stent to fit and move more easily within a constrained space in a catheter, by reducing localized buckling and radial expansion of the self-expanding stent. The system includes features for moving the self-expanding stent back and/or forth inside a catheter preferentially from one or more of a proximal end, distal end or some other specific location therebetween of the self-expanding stent, essentially by allowing the self-expanding stent to be moved distally and proximally by dragging or pulling the self-expanding stent from distal and/or proximal portions, respectively, instead of by pushing the proximal end of the self-expanding stent distally or pushing the distal end of the self-expanding stent proximally, and thereby reducing the force needed to drive the device forward distally and/or rearwardly proximally.
The present invention is defined by the independent claim. Further details of specific embodiments are given in the dependent claims.

Other features and advantages of the present invention will become more apparent from the following detailed description of the preferred embodiments in conjunction with the accompanying drawings, which illustrate, by way of example, the operation of the invention.

### BRIEF DESCRIPTION OF THE DRAWINGS

Figure 1 is an enlarged partial cross-sectional schematic diagram of a system for delivering and releasing a self-expanding stent to a treatment site in a patient's vasculature, according to the invention.
Fig. 2 is an enlarged partial cross-sectional schematic diagram similar to Fig. 1, showing the system disposed within a blood vessel and aligned adjacent to a treatment site in a patient's vasculature.
Fig. 3 is an enlarged partial cross-sectional schematic diagram similar to Fig. 2, with a portion of the self-expanding stent cut away to expose radiopaque markers or stop members on the core advancement wire.
Fig. 4 is an enlarged partial cross-sectional schematic diagram similar to Fig. 3, illustrating pushing the device distally.
Fig. 5 is an enlarged partial cross-sectional schematic diagram similar to Fig. 4, illustrating pulling the device proximally.
Fig. 6 is an enlarged partial sectional view of a deployment catheter moved proximally with a proximal portion of the self-expanding stent compressed within a deployment catheter and a distal portion of the self-expanding stent expanded within the patient's vasculature.
Fig. 7 is a cross-sectional elevational schematic diagram illustrating an expanded configuration of the self-expanding stent of Fig. 1 within the patient's vasculature.
Fig. 8 is a cross-sectional elevational schematic diagram of a variation of the system of Fig. 1, with a portion of the self-expanding stent cut away to expose radiopaque markers or stop members on the core advancement wire.
Fig. 9 is an enlarged partial sectional view of the self-expanding stent and delivery system disposed within a patient's vasculature and aligned adjacent to an aneurysm.
Fig. 10 is an enlarged partial sectional view of the deployment catheter moved proximally, with the proximal portion of the self-expanding stent constrained within the deployment catheter and the distal portion of the self-expanding stent expanded within the patient's vasculature.
Fig. 11 is an enlarged sectional view of the self-expanding stent expanded within the patient's vasculature and covering a mouth of the aneurysm.
Fig. 12 is an enlarged sectional view of the stent expanded within the vessel and a microcatheter inserted through the wall of the self-expanding stent and into the aneurysm.
Fig. 13 is an enlarged sectional view of the self-expanding stent expanded within the patient's vasculature and covering the mouth of the aneurysm with an embolic coil deployed within the aneurysm.

### DETAILED DESCRIPTION OF THE PREFERRED EMBODIMENTS

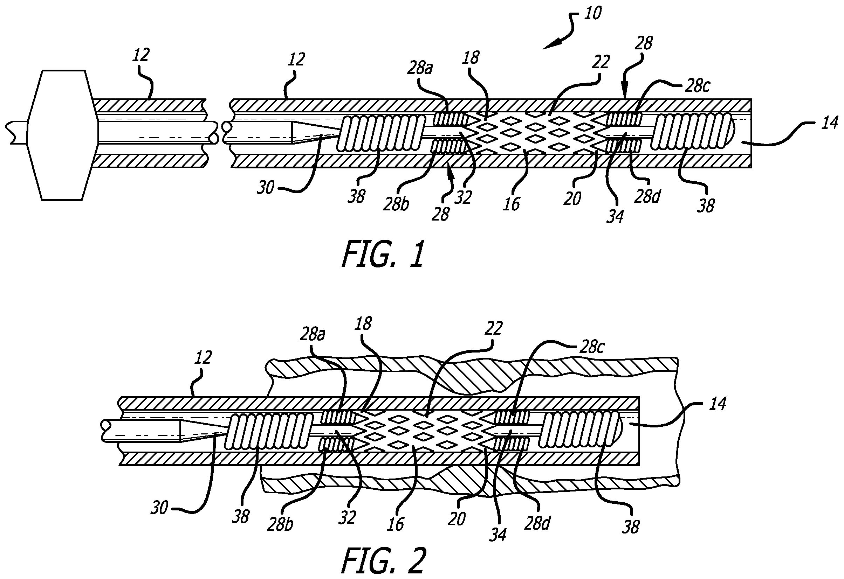

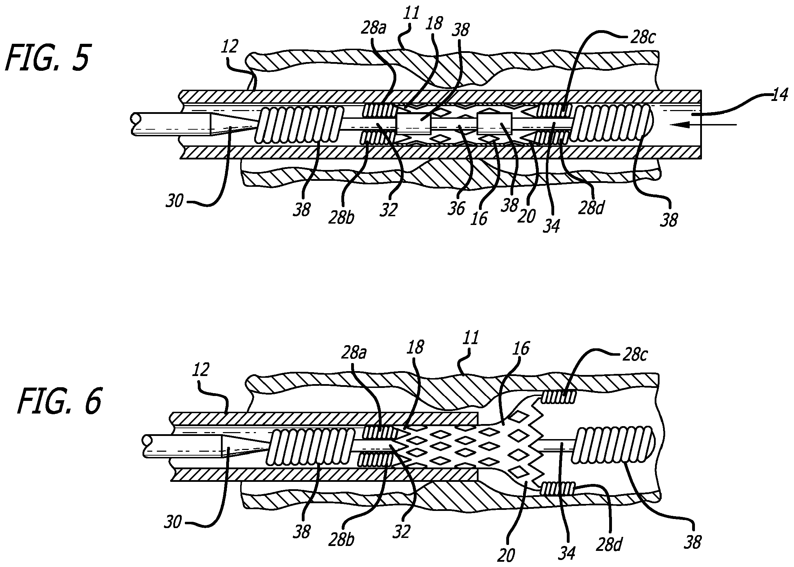

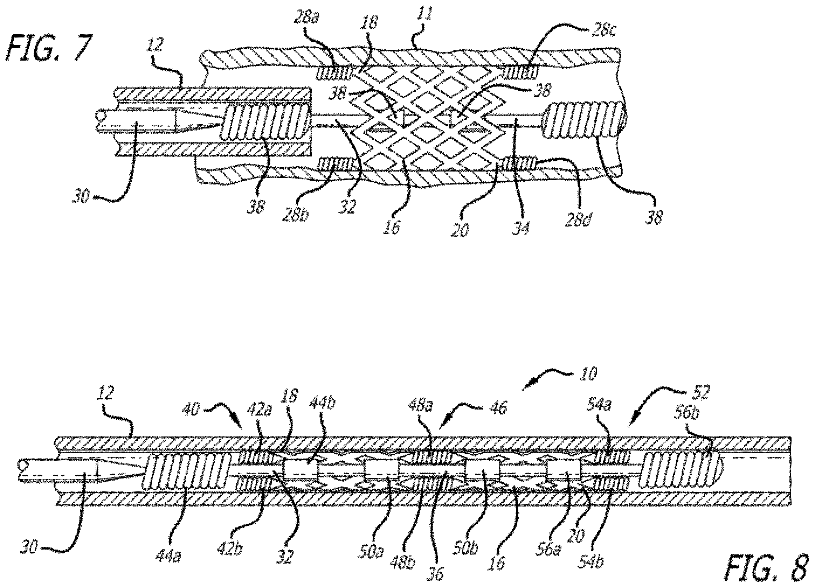

Referring to the drawings, which are provided by way of example, and not by way of limitation, the present invention provides for an apparatus 10 for delivering and releasing a self-expanding stent to a treatment site in a patient's vasculature 11. As is illustrated in Fig. 1, the apparatus includes a catheter 12 having an inner lumen 14, a tubular self-expanding stent 16 having an inner lumen (not shown), a proximal end 18, a distal end 20, and an intermediate portion 22 located between the proximal end and the distal end, and an inner lumen (not shown). Referring to Figs. 1-5, the tubular self-expanding stent has a compressed configuration dimensioned to fit within the inner lumen of the catheter, such that the tubular self-expanding stent is configured to be constrained from expanding when the tubular self-expanding stent contained within the catheter, and an expanded configuration, illustrated in Fig. 7. The tubular self-expanding stent preferably includes one or more anchor members 28, such as proximal anchor members 28a, 28b disposed on opposing sides of the core advancement wire, for example, at the proximal end of the tubular self-expanding stent, and distal anchor members 28c, 28d disposed on opposing sides of the core advancement wire, for example, at the distal end of the tubular self-expanding stent, connected to and extending radially inwardly from the tubular self-expanding stent, as will be explained further below.

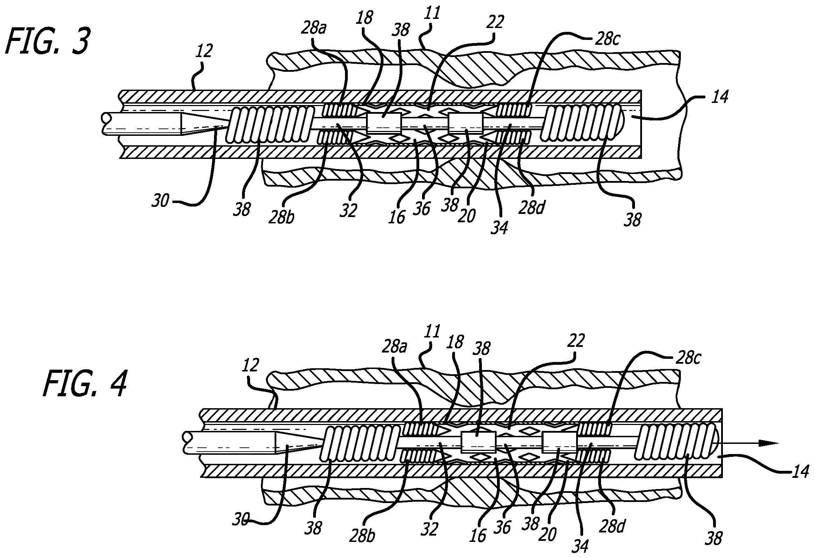

A core advancement wire 30 is disposed within and extends through the lumen of the tubular stent. The core advancement wire has a proximal portion 32, a distal portion 34, and an intermediate portion 36 (shown in Figs. 3-5 and 7) located between the proximal and distal portions of the core advancement wire. The core advancement wire advantageously includes one or more radiopaque markers or stop members 38 extending radially outwardly from the core advancement wire and configured to engage the one or more anchor members when the core advancement wire is translated longitudinally toward the one or more anchor members, whereby force applied longitudinally to the core advancement wire is transmitted through the one or more anchor members to the tubular self-expanding stent and acts to move the tubular self-expanding stent through the catheter when the stent is constrained within the catheter. In a presently preferred aspect, the one or more radiopaque markers or stop members have a diameter greater than or equal to an inward radial extension of the one or more anchor members when the tubular self-expanding stent is in the compressed configuration.

In one presently preferred aspect, the one or more radiopaque markers or stop members can be formed as a sleeve or a coil made of polymer or metal, for example, and fixedly attached to the core advancement wire. In another presently preferred aspect, the one or more radiopaque markers or stop members can be formed as a ground step profile formed on the core advancement wire, or as an enlarged portion of the core advancement wire having a diameter greater than or equal to an inward radial extension of the one or more anchor members when the tubular self-expanding stent is in the compressed configuration.

Referring to Fig. 8, the one or more anchor members can for example include one or more proximal anchor members 40, such as two proximal anchor members 42a, 42b disposed on opposing sides of the core advancement wire, for example, and disposed at or near the proximal end of the tubular self-expanding stent, extending radially outwardly of the core advancement wire. The one or more proximal anchor members are configured to be engaged by the one or more proximal radiopaque markers or stop members, such as first and second proximal radiopaque markers or stop members 44a, 44b spaced apart from each other, with the one or more proximal anchor members disposed between the first and second proximal radiopaque markers or stop members.

In another presently preferred aspect, the one or more anchor members can for example also include one or more intermediate anchor members 46, such as two intermediate anchor members 48a, 48b disposed on opposing sides of the core advancement wire, for example, and disposed at or near the intermediate portion of the tubular self-expanding stent, extending radially outwardly of the core advancement wire and configured to be engaged by the one or more intermediate radiopaque markers or stop members, such as first and second intermediate radiopaque markers or stop members 50a, 50b spaced apart from each other, with the one or more intermediate anchor member disposed between the first and second intermediate radiopaque markers or stop members.

The one or more anchor members can for example also include one or more distal anchor members 52, such as two distal anchor members 54a, 54b disposed on opposing sides of the core advancement wire, for example, and disposed at or near the distal end of the tubular self-expanding stent, extending radially outwardly of the core advancement wire. The one or more distal anchor members are configured to be engaged by one or more distal radiopaque markers or stop members, such as first and second distal radiopaque markers or stop members 56a, 56b spaced apart from each other, with the one or more distal anchor members disposed between the first and second distal radiopaque markers or stop members, when the core advancement wire is translated longitudinally toward the one or more distal anchor members.

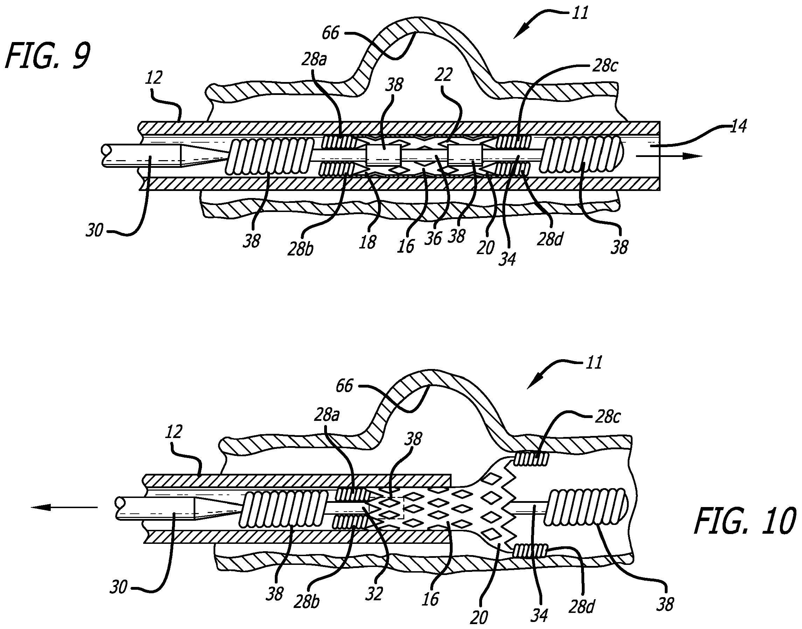

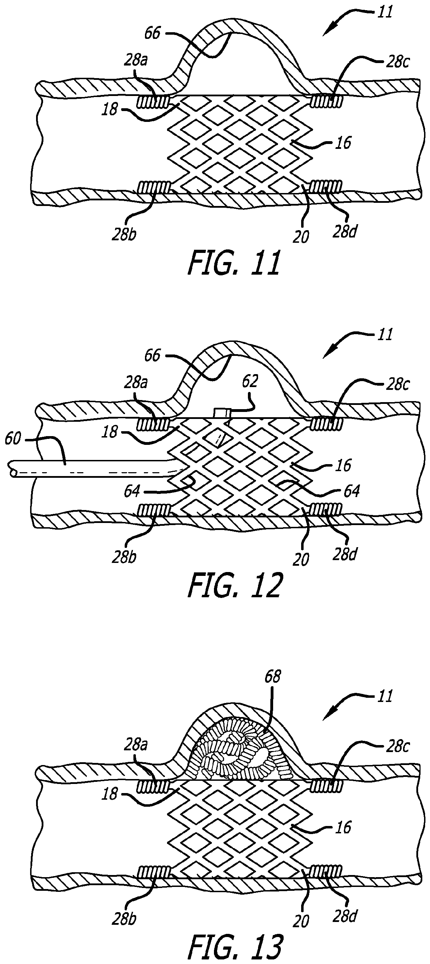

Referring to Fig. 9, the catheter and self-expanding stent can, for example, be positioned at a treatment site within a patient's vasculature and aligned across a mouth of an aneurysm 66. As is shown in The self-expanding stent can be at first only partially deployed within the patient's vasculature, affording the opportunity of retracting the self-expanding stent proximally within the catheter, as is illustrated in Fig. 10, such as for later repositioning and deployment of the self-expanding stent. The self-expanding stent can be fully deployed by moving the self-expanding stent distally and withdrawing the catheter proximally, causing the anchor members on the distal end of the self-expanding stent to anchor the distal end of the self-expanding stent to allow the self-expanding stent to deploy to cover the mouth of the aneurysm, as is illustrated in Fig. 11.

Referring to Fig. 12, once the self-expanding stent is deployed cover the mouth of the aneurysm, a microcatheter 60 can be inserted into the patient's vasculature, and a distal portion 62 of the microcatheter may be insert through one of the interstices 64 of the self-expanding stent, and into the aneurysm, so that one or more embolic coils 68 or other embolic agents can be delivered into the aneurysm, as is illustrated in Fig. 13.

It will be apparent from the foregoing that while particular forms of the invention have been illustrated and described, various modifications can be made without departing from the scope of the invention. Accordingly, it is not intended that the invention be limited, except as by the appended claims.

## Claims

1. A system for delivering and releasing a self-expanding stent to a treatment site in a patient's vasculature, said system comprising:
a catheter (12) having an inner lumen (14);
a tubular self-expanding stent (16) having an inner lumen, a proximal end (18), a distal end (20), and an intermediate portion (22) located between said proximal end and said distal end, said tubular self-expanding stent having a compressed configuration dimensioned to fit within said inner lumen of said catheter and an expanded configuration, said tubular self-expanding stent being configured to be constrained from expanding when said tubular self-expanding stent contained within said catheter, and said tubular self-expanding stent including at least one anchor member (28) extending radially inwardly into said inner lumen; and
a core advancement wire (30) disposed within and extending through said lumen of said tubular stent, said core advancement wire having a proximal portion (32), a distal portion (34), an intermediate portion (36) located between said proximal and distal portions of said core advancement wire, and said core advancement wire having at least one stop member (38) extending radially outwardly from said core advancement wire and configured to engage said at least one anchor member when said core advancement wire is translated longitudinally toward said at least one anchor member, whereby force applied longitudinally to said core advancement wire is transmitted through said at least anchor member to said tubular self-expanding stent and acts to move said tubular self-expanding stent through said catheter when said stent is constrained within said catheter;
wherein said at least one anchor member comprises at least one proximal anchor member (40) disposed at or near said proximal end of said tubular self-expanding stent, and at least one distal anchor member (54a,54b) disposed at or near said distal end of said tubular self-expanding stent; and
wherein said at least one stop member comprises at least one proximal cylindrical stop member within the inner lumen of the stent extending radially outwardly from said core advancement wire and configured to engage said at least one proximal anchor member when said core advancement wire is translated longitudinally toward said at least one proximal anchor member, and at least one distal cylindrical stop member (56a,56b) within the inner lumen of the stent extending radially outwardly from said core advancement wire and configured to engage said at least one distal anchor member when said core advancement wire is translated distally longitudinally toward said at least one distal anchor member.

2. The system of Claim 1, wherein said at least one proximal anchor member comprises two proximal anchor members (42a, 42b) disposed on opposing sides of said core advancement wire.

3. The system of Claim 1, wherein said at least one stop member (38) comprises first and second proximal stop members (44a,44b) spaced apart from each other, and said at least one proximal anchor member is disposed between said first and second proximal stop members.

4. The system of Claim 2, wherein said at least one stop member comprises first and second proximal stop members (54a,54b) spaced apart from each other, and said two proximal anchor members are disposed between said first and second proximal stop members.

5. The system of Claim 1, wherein said at least one distal anchor member comprises two distal anchor members (54a,54b) disposed on opposing sides of said core advancement wire.

6. The system of Claim 1, wherein said at least one stop member comprises first and second distal stop members (56a,56b) spaced apart from each other, and said at least one distal anchor member (52) is disposed between said first and second distal stop members.

7. The system of Claim 5, wherein said at least one stop member comprises first and second distal stop members (56a,56b) spaced apart from each other, and said two distal anchor members are disposed between said first and second distal stop members.

8. The system of Claim 1, wherein said at least one anchor member comprises at least one intermediate anchor member (48a,48b) disposed at or near said intermediate portion of said tubular self-expanding stent, and said at least one stop member comprises at least one intermediate stop member (50a,50b) extending radially outwardly from said core advancement wire and configured to engage said at least one intermediate anchor member when said core advancement wire is translated longitudinally toward said at least one intermediate anchor member.

9. The system of Claim 8, wherein said at least one intermediate anchor member (48a,48b) comprises two intermediate anchor members disposed on opposing sides of said core advancement wire.

10. The system of Claim 8, wherein said at least one stop member comprises first and second intermediate stop members (50a,50b)spaced apart from each other, and said at least one intermediate anchor member is disposed between said first and second intermediate stop members.

11. The system of Claim 9, wherein said at least one stop member comprises first and second intermediate stop members (50a,50b) spaced apart from each other, and said two intermediate anchor members are disposed between said first and second intermediate stop members.

12. The system of Claim 1, wherein said at least one stop member (38) has a diameter greater than or equal to an inward radial extension of said at least one anchor member (28) when said tubular self-expanding stent is in said compressed configuration.

13. The system of Claim 1, wherein said at least one stop member (38) comprises a sleeve fixedly attached to said core advancement wire, a coil fixedly attached to said core advancement wire, or a ground step profile formed on said core advancement wire, or
wherein said at least one stop member (38)comprises an enlarged portion of said core advancement wire having a diameter greater than or equal to an inward radial extension of said at least one anchor member when said tubular self-expanding stent is in said compressed configuration.

## Patentansprüche

1. System zum Abgeben und Freigeben eines selbstexpandierenden Stents an eine Behandlungsstelle in dem Gefäßsystem eines Patienten, wobei das System umfasst:
einen Katheter (12) mit einem inneren Lumen (14);
einen rohrförmigen selbstexpandierenden Stent (16) mit einem inneren Lumen, einem proximalen Ende (18), einem distalen Ende (20) und einem mittleren Teil (22), der zwischen dem proximalen Ende und dem distalen Ende angeordnet ist, wobei der rohrförmige selbstexpandierende Stent eine komprimierte Konfiguration, die bemessen ist, in das innere Lumen des Katheters zu passen, und eine expandierte Konfiguration aufweist, wobei der rohrförmige selbstexpandierende Stent dafür gestaltet ist, vom Expandieren zurückgehalten zu werden, wenn der rohrförmige selbstexpandierende Stent in dem Katheter enthalten ist, und der rohrförmige selbstexpandierende Stent wenigstens ein Ankerelement (28) aufweist, das radial nach innen in das innere Lumen ragt; und
einen Kern-Vorschubdraht (30), der innerhalb des Lumens des rohrförmigen Stents angeordnet ist und hindurch verläuft, wobei der Kern-Vorschubdraht einen proximalen Teil (32), einen distalen Teil (34), einen mittleren Teil (36), der zwischen dem proximalen und dem distalen Teil des Kern-Vorschubdrahts angeordnet ist, aufweist, und der Kern-Vorschubdraht wenigstens ein Anschlagelement (38) aufweist, das von dem Kern-Vorschubdraht radial nach außen ragt und dafür gestaltet ist, mit dem wenigstens einen Ankerelement in Eingriff zu kommen, wenn der Kern-Vorschubdraht in Längsrichtung in Richtung zu dem wenigstens einen Ankerelement verschoben wird, wodurch Kraft, die in Längsrichtung auf den Kern-Vorschubdraht ausgeübt wird, durch das wenigstens eine Ankerelement auf den rohrförmigen selbstexpandierenden Stent übertragen wird und wirkt, den rohrförmigen selbstexpandierenden Stent durch den Katheter zu bewegen, wenn der Stent innerhalb des Katheters eingeschlossen ist;
wobei das wenigstens eine Ankerelement wenigstens ein proximales Ankerelement (40), das an oder nahe dem proximalen Ende des rohrförmigen selbstexpandierenden Stents angeordnet ist, und wenigstens ein distales Ankerelement (54a, 54b), das an oder nahe dem distalen Ende des rohrförmigen selbstexpandierenden Stents angeordnet ist, umfasst; und
wobei das wenigstens eine Anschlagelement wenigstens ein proximales zylindrisches Anschlagelement innerhalb des inneren Lumens des Stents umfasst, das von dem Kern-Vorschubdraht radial nach außen ragt und dafür gestaltet ist, mit dem wenigstens einen proximalen Ankerelement in Eingriff zu kommen, wenn der Kern-Vorschubdraht in Längsrichtung in Richtung zu dem wenigstens einen proximalen Ankerelement verschoben wird, und wenigstens ein distales zylindrisches Anschlagelement (56a, 56b) innerhalb des inneren Lumens des Stents, das von dem Kern-Vorschubdraht radial nach außen ragt und dafür gestaltet ist, mit dem wenigstens einen distalen Ankerelement in Eingriff zu kommen, wenn der Kern-Vorschubdraht in Längsrichtung distal in Richtung zu dem wenigstens einen distalen Ankerelement verschoben wird.

2. System gemäß Anspruch 1, wobei das wenigstens eine proximale Ankerelement zwei proximale Ankerelemente (42a, 42b) umfasst, die an gegenüberliegenden Seiten des Kern-Vorschubdrahts angeordnet sind.

3. System gemäß Anspruch 1, wobei das wenigstens eine Anschlagelement (38) ein erstes und ein zweites proximales Anschlagelement (44a, 44b) umfasst, die voneinander beabstandet sind, und das wenigstens eine proximale Ankerelement zwischen dem ersten und dem zweiten proximalen Anschlagelement angeordnet ist.

4. System gemäß Anspruch 2, wobei das wenigstens eine Anschlagelement ein erstes und ein zweites proximales Anschlagelement (54a, 54b) umfasst, die voneinander beabstandet sind, und die beiden proximalen Ankerelemente zwischen dem ersten und dem zweiten proximalen Anschlagelement angeordnet sind.

5. System gemäß Anspruch 1, wobei das wenigstens eine distale Ankerelement zwei distale Ankerelemente (54a, 54b) umfasst, die an gegenüberliegenden Seiten des Kern-Vorschubdrahts angeordnet sind.

6. System gemäß Anspruch 1, wobei das wenigstens eine Anschlagelement ein erstes und ein zweites distales Anschlagelement (56a, 56b) umfasst, die voneinander beabstandet sind, und das wenigstens eine distale Ankerelement (52) zwischen dem ersten und dem zweiten distalen Anschlagelement angeordnet ist.

7. System gemäß Anspruch 5, wobei das wenigstens eine Anschlagelement ein erstes und ein zweites distales Anschlagelement (56a, 56b) umfasst, die voneinander beabstandet sind, und die beiden distalen Ankerelemente zwischen dem ersten und dem zweiten distalen Anschlagelement angeordnet sind.

8. System gemäß Anspruch 1, wobei das wenigstens eine Ankerelement wenigstens ein mittleres Ankerelement (48a, 48b) umfasst, das an oder nahe dem mittleren Teil des rohrförmigen selbstexpandierenden Stents angeordnet ist, und das wenigstens eine Anschlagelement wenigstens ein mittleres Anschlagelement (50a, 50b) umfasst, das von dem Kern-Vorschubdraht radial nach außen ragt und dafür gestaltet ist, mit dem wenigstens einen mittleren Ankerelement in Eingriff zu kommen, wenn der Kern-Vorschubdraht in Längsrichtung in Richtung zu dem wenigstens einen mittlere Ankerelement verschoben wird.

9. System gemäß Anspruch 8, wobei das wenigstens eine mittlere Ankerelement (48a, 48b) zwei mittlere Ankerelemente umfasst, die an gegenüberliegenden Seiten des Kern-Vorschubdrahts angeordnet sind.

10. System gemäß Anspruch 8, wobei das wenigstens eine Anschlagelement ein erstes und ein zweites mittleres Anschlagelement (50a, 50b) umfasst, die voneinander beabstandet sind, und das wenigstens eine mittlere Ankerelement zwischen dem ersten und dem zweiten mittleren Anschlagelement angeordnet ist.

11. System gemäß Anspruch 9, wobei das wenigstens eine Anschlagelement ein erstes und ein zweites mittleres Anschlagelement (50a, 50b) umfasst, die voneinander beabstandet sind, und die beiden mittleren Ankerelemente zwischen dem ersten und dem zweiten mittleren Anschlagelement angeordnet sind.

12. System gemäß Anspruch 1, wobei das wenigstens eine Anschlagelement (38) einen Durchmesser aufweist, der größer als oder gleich einer inneren radialen Ausdehnung des wenigstens einen Ankerelements (28) ist, wenn der rohrförmige selbstexpandierende Stent in der komprimierten Konfiguration vorliegt.

13. System gemäß Anspruch 1, wobei das wenigstens eine Anschlagelement (38) eine Hülse, die fest an dem Kern-Vorschubdraht befestigt ist, eine Spule, die fest an dem Kern-Vorschubdraht befestigt ist, oder ein geschliffenes Stufenprofil, das an dem Kern-Vorschubdraht gebildet ist, umfasst, oder
wobei das wenigstens eine Anschlagelement (38) einen vergrößerten Teil des Kern-Vorschubdrahts umfasst, der einen Durchmesser aufweist, der größer als oder gleich einer inneren radialen Ausdehnung des wenigstens einen Ankerelements ist, wenn der rohrförmige selbstexpandierende Stent in der komprimierten Konfiguration vorliegt.

## Revendications

1. Système de délivrance et de libération d'un stent auto-expansible sur un site de traitement dans le système vasculaire d'un patient, ledit système comprenant :
un cathéter (12) présentant une lumière intérieure (14) ;
un stent tubulaire auto-expansible (16) présentant une lumière intérieure, une extrémité proximale (18), une extrémité distale (20), et une partie intermédiaire (22) située entre ladite extrémité proximale et ladite extrémité distale, ledit stent tubulaire auto-expansible présentant une configuration comprimée dimensionnée de manière à s'ajuster à l'intérieur de ladite lumière intérieure dudit cathéter et une configuration expansée, ledit stent tubulaire auto-expansible étant configuré de manière à être contraint pour ne pas s'expanser lorsque ledit stent tubulaire auto-expansible est contenu à l'intérieur dudit cathéter, et ledit stent tubulaire auto-expansible comportant au moins un élément d'ancrage (28) s'étendant radialement vers l'intérieur dans ladite lumière intérieure ; et
un fil d'avance central (30) disposé à l'intérieur et s'étendant à travers ladite lumière dudit stent tubulaire, ledit fil d'avance central présentant une partie proximale (32), une partie distale (34), une partie intermédiaire (36) située entre lesdites parties proximale et distale dudit fil d'avance central, et ledit fil d'avance central présentant au moins un élément d'arrêt (38) s'étendant radialement vers l'extérieur à partir dudit fil d'avance central et configuré de manière à s'enclencher avec ledit au moins un élément d'ancrage lorsque ledit fil d'avance central est translaté longitudinalement vers ledit au moins un élément d'ancrage, moyennant quoi une force appliquée longitudinalement audit fil d'avance central est transmise à travers ledit au moins un élément d'ancrage audit stent tubulaire auto-expansible et agit pour déplacer ledit stent tubulaire auto-expansible à travers ledit cathéter lorsque ledit stent est contraint à l'intérieur dudit cathéter ;
dans lequel ledit au moins un élément d'ancrage comprend au moins un élément d'ancrage proximal (40) disposé à ou à proximité de ladite extrémité proximale dudit stent tubulaire auto-expansible, et au moins un élément d'ancrage distal (54a, 54b) disposé à ou à proximité de ladite extrémité distale dudit stent tubulaire auto-expansible ; et
dans lequel ledit au moins un élément d'arrêt comprend au moins un élément d'arrêt cylindrique proximal à l'intérieur de la lumière intérieure du stent s'étendant radialement vers l'extérieur à partir dudit fil d'avance central et configuré pour s'enclencher avec ledit au moins un élément d'ancrage proximal lorsque ledit fil d'avance central est translaté longitudinalement vers ledit au moins un élément d'ancrage proximal, et au moins un élément d'arrêt cylindrique distal (56a, 56b) à l'intérieur de la lumière intérieure du stent s'étendant radialement vers l'extérieur à partir dudit fil d'avance central et configuré pour s'enclencher avec ledit au moins un élément d'ancrage distal lorsque ledit fil d'avance central est translaté de façon distale longitudinalement vers ledit au moins un élément d'ancrage distal.

2. Système de la revendication 1, dans lequel ledit au moins un élément d'ancrage proximal comprend deux éléments d'ancrage proximaux (42a, 42b) disposés sur des côtés opposés dudit fil d'avance central.

3. Système de la revendication 1, dans lequel ledit au moins un élément d'arrêt (38) comprend des premier et deuxième éléments d'arrêt proximaux (44a, 44b) espacés l'un de l'autre, et ledit au moins un élément d'ancrage proximal est disposé entre lesdits premier et deuxième éléments d'arrêt proximaux.

4. Système de la revendication 2, dans lequel ledit au moins un élément d'arrêt comprend des premier et deuxième éléments d'arrêt proximaux (54a, 54b) espacés l'un de l'autre, et lesdits deux éléments d'ancrage proximaux sont disposés entre lesdits premier et deuxième éléments d'arrêt proximaux.

5. Système de la revendication 1, dans lequel ledit au moins un élément d'ancrage distal comprend deux éléments d'ancrage distaux (54a, 54b) disposés sur des côtés opposés dudit fil d'avance central.

6. Système de la revendication 1, dans lequel ledit au moins un élément d'arrêt comprend des premier et deuxième éléments d'arrêt distaux (56a, 56b) espacés l'un de l'autre, et ledit au moins un élément d'ancrage distal (52) est disposé entre lesdits premier et deuxième éléments d'arrêt distaux.

7. Système de la revendication 5, dans lequel ledit au moins un élément d'arrêt comprend des premier et deuxième éléments d'arrêt distaux (56a, 56b) espacés l'un de l'autre, et lesdits deux éléments d'ancrage distaux sont disposés entre lesdits premier et deuxième éléments d'arrêt distaux.

8. Système de la revendication 1, dans lequel ledit au moins un élément d'ancrage comprend au moins un élément d'ancrage intermédiaire (48a, 48b) disposé dans ou à proximité de ladite partie intermédiaire dudit stent tubulaire auto-expansible, et ledit au moins un élément d'arrêt comprend au moins un élément d'arrêt intermédiaire (50a, 50b) s'étendant radialement vers l'extérieur à partir dudit fil d'avance central et configuré pour s'enclencher avec ledit au moins un élément d'ancrage intermédiaire lorsque ledit fil d'avance central est translaté longitudinalement vers ledit au moins un élément d'ancrage intermédiaire.

9. Système de la revendication 8, dans lequel ledit au moins un élément d'ancrage intermédiaire (48a, 48b) comprend deux éléments d'ancrage intermédiaires disposés sur des côtés opposés dudit fil d'avance central.

10. Système de la revendication 8, dans lequel ledit au moins un élément d'arrêt comprend des premier et deuxième éléments d'arrêt intermédiaires (50a, 50b) espacés l'un de l'autre, et ledit au moins un élément d'ancrage intermédiaire est disposé entre lesdits premier et deuxième éléments d'arrêt intermédiaires.

11. Système de la revendication 9, dans lequel ledit au moins un élément d'arrêt comprend des premier et deuxième éléments d'arrêt intermédiaires (50a, 50b) espacés l'un de l'autre, et lesdits deux éléments d'ancrage intermédiaires sont disposés entre lesdits premier et deuxième éléments d'arrêt intermédiaires.

12. Système de la revendication 1, dans lequel ledit au moins un élément d'arrêt (38) a un diamètre supérieur ou égal à une extension radiale intérieure dudit au moins un élément d'ancrage (28) lorsque ledit stent tubulaire auto-expansible se trouve dans ladite configuration comprimée.

13. Système de la revendication 1, dans lequel ledit au moins un élément d'arrêt (38) comprend un manchon attaché de façon fixe audit fil d'avance central, une bobine attachée de façon fixe audit fil d'avance central, ou un profil de marches meulées formé sur ledit fil d'avance central, ou
dans lequel ledit au moins un élément d'arrêt (38) comprend une partie agrandie dudit fil d'avance central présentant un diamètre supérieur ou égal à une extension radiale intérieure dudit au moins un élément d'ancrage lorsque ledit stent tubulaire auto-expansible se trouve dans ladite configuration comprimée.
